# EUROPEAN PATENT SPECIFICATION

(11) **EP 0 402 407 B1**
(45) Date of publication and mention of the grant of the patent: **03.08.1994**
(21) Application number: 89903563.8
(22) Date of filing: 27.02.1989
(51) Int. Cl.: A61L 15/16

(54) **TRANSDERMAL ESTRADIOL DELIVERY SYSTEM**
TRANSDERMALES ESTRADIOL ALS THERAPEUTISCHES SYTEM
SYSTEME D'ADMINISTRATION TRANSCUTANEE D'ESTRADIOL

(30) Priority: 26.02.1988 US 160635
(43) Date of publication of application: 19.12.1990
(73) Proprietor: RIKER LABORATORIES, INC., St. Paul, MN 55144 (US)
(72) Inventor: NELSON, Gregory R., St. Paul, MN 55133-3427 (US); ZERBE, Horst-Georg, D-4282 Velen (DE); MOORE, Cheryl L., St. Paul, MN 55133-3427 (US); WICK, Steven M., St. Paul, MN 55133-3427 (US)
(74) Representative: Madgwick, Paul Roland
(86) International application number: US8900786
(87) International publication number: WO8907951

(56) References cited:
- EP-A- 0 062 682
- EP-A- 0 156 080
- EP-A- 0 272 987
- EP-A- 0 279 986
- WO-A-86/06281
- GB-A- 2 086 224
- US-A- 4 573 996

## Description

### Technical Field

This invention relates to a pressure-sensitive adhesive sheet material containing estradiol in the adhesive portion of the sheet material. This invention further relates to a method of treating conditions associated with estradiol deficiency, such as osteoporosis and headaches, nausea, depression, hot flashes or other discomforts that often occur during menopause.

### Background of the Invention

Estradiol is a natural estrogen which has limited oral effectiveness because it is rapidly metabolized by the liver to estrone and its conjugates, giving rise to higher circulating levels of estrone than estradiol. In contrast, the skin metabolizes estradiol only to a small extent. Therefore, transdermal administration produces therapeutic serum levels of estradiol with lower circulating levels of estrone and estrone conjugates, and requires smaller total doses than does oral therapy. Since estradiol has a short half-life (about one hour), transdermal administration of estradiol allows a rapid decline in blood levels after a transdermal system is removed.

Estraderm^{R} is an estradiol transdermal system available from CIBA Pharmaceutical Company. This system comprises four layers: a transparent polyester film; a drug reservoir of estradiol and alcohol gel with hydroxypropyl cellulose; an ethylene-vinylacetate copolymer membrane; and an adhesive formulation of light mineral oil and polyisobutylene for adhering the patch to skin.

Japanese Application 57075917 describes the manufacture of a tacky tape for use with a variety of sex hormones including valeric acid-estradiol. The tape is prepared by (1) copolymerizing (a) 60-98 parts by weight of dodecyl methacrylate, (b) 2-40 pts. wt. of a functional monomer and (c) 0-40 pts. wt. of at least one short chain unsaturated monomer selected from vinyl acetate, an alkyl acrylate, and an alkyl methacrylate; (2) combining the drug with the copolymer; and (3) spreading the resulting composition onto base material.

U. S. Patent 3,598,123 describes a medical bandage for use with a variety of drugs including estradiol. The bandage comprises: a backing member and a layer of pressure-sensitive adhesive containing a plurality of discrete microcapsules containing the drug. Acrylic adhesives are specifically mentioned.

U.S. Patent No. 4,460,372 describes a transdermal device comprising a backing and an adhesive layer, the adhesive layer containing both estradiol and a microencapsulated percutaneous absorption enhancer such as ethanol.

GB Application 2158355 describes an estradiol containing transdermal dosage form comprising: a solid non-polymeric gel; a mixture of propylene glycol and glycerin; the therapeutic agent dispersed in the solvent mixture; and a thin, flexible, non-polymeric matrix in planar form. The mixture of propylene glycol and glycerine is described as enhancing the skin penetration of the therapeutic agent.

U.S. Patent Nos. 3,598,122, 4,379,452, 4,573,996, 4,585,454, 4,624,665, and 4,460,372 (also mentioned above) all describe estradiol transdermal patches which include layers in addition to a backing and an adhesive layer. For example, many of these patents describe patches comprising a backing, a drug reservoir layer; a semipermeable membrane; and an adhesive layer coated on the exterior surface of the semipermeable membrane. Said U.S. Patent No. 4,573,996 discloses a variety of penetration enhancers in Col. 11, lines 44-68.

European Application Publication Number 0219539 describes a transdermal nitroglycerin delivery system comprising a flexible backing and a pressure-sensitive adhesive coating comprising an acrylic polymer and nitroglycerin. The adhesive coating may also comprise a skin penetration enhancing combination comprising a fatty acid ester of a fatty acid and glyceryl monolaurate.

U.S. Patent No. 4,722,941 discloses transdermal and oral formulations employing a fatty acid of medium chain length and optionally a monoglyceride of a saturated or unsaturated fatty acid of about 6 to 18 carbon atoms to enhance drug absorption. The formulations may include a steroid.

Glyceryl monolaurate, isopropyl myristate and ethyl oleate are known enhancers for transdermal administration of medicaments.

### BRIEF SUMMARY OF THE INVENTION

The present invention provides a novel adhesive-coated sheet material comprising:
a) a flexible backing; and
b) a pressure-sensitive adhesive-coating contiguously adhered to one surface of said backing and comprising a homogeneous mixture of:
   i) an acrylic polymer comprising at least 91 to 98 percent by weight of a hydrophobic monomeric acrylic or methacrylic acid ester of an alkyl alcohol based on the weight of all monomers in the polymer, the alkyl alcohol containing 4 to 10 carbon atoms;
   ii) estradiol in an amount by weight of 0.2 to 12 percent of the total weight of the adhesive coating; and
   iii) a skin penetration enhancer combination comprising isopropyl myristate and glyceryl monolaurate in amounts of 5 to 20 percent and 1 to 6 percent by weight, respectively, based on the weight of the adhesive-coating with the relative amounts being selected so as to enhance the penetration of the estradiol through skin as compared to when the adhesive coating is free of said skin penetration enhancers;
the sheet material being suitable for substantially continuous transdermal delivery of estradiol to a subject over a prolonged period in an amount which is therapeutically effective for treating a condition associated with estradiol deficiency.

The present invention also provides a novel adhesive-coated sheet material comprising:
a) a flexible backing; and
b) a pressure-sensitive adhesive-coating contiguously adhered to one surface of said backing and comprising a homogeneous mixture of:
   i) an acrylic copolymer comprising (1) 60 to 80 percent by weight of a hydrophobic monomeric acrylic or methacrylic acid ester of an alkyl alcohol based on the weight of all of the monomers in the copolymer, the alkyl alcohol containing 4 to 10 carbon atoms; (2) 4 to 9 percent by weight based on the weight of all of the monomers in the copolymer of a reinforcing monomer selected from the group consisting of acrylic acid, methacrylic acid, an alkyl acrylate or methacrylate containing 1 to 3 carbon atoms in the alkyl group, acrylamide, methacrylamide, a lower alkyl-substituted acrylamide, diacetone acrylamide, and a N-vinyl-2-pyrrolidone; and (3) 15 to 35 percent by weight of vinyl acetate based on the weight of all of the monomers in the copolymer;
   ii) estradiol in an amount by weight of 0.2 to 12 percent of the total weight of the adhesive coating; and
   iii) a skin penetration enhancer combination comprising isopropyl myristate and glyceryl monolaurate in amounts of 5 to 20 percent and 1 to 6 percent by weight, respectively, based on the weight of the adhesive coating, with the relative amounts being selected so as to enhance the penetration of the estradiol through skin as compared to when the adhesive coating is free of the skin penetration enhancers;
the sheet material being suitable for substantially continuous transdermal delivery of estradiol to a subject over a prolonged period in an amount which is therapeutically effective for treating a condition associated with estradiol deficiency.

In preferred embodiments of the invention, the skin penetration enhancer combination further contains ethyl oleate.

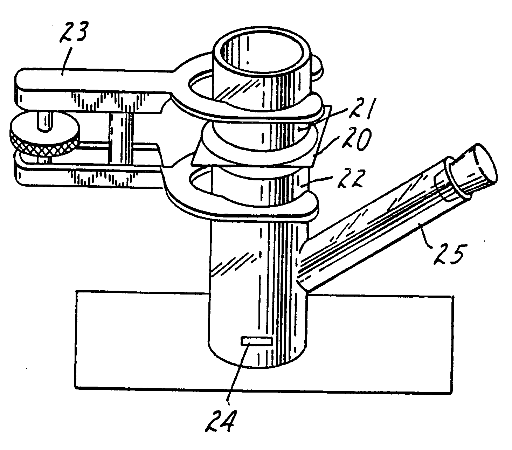

### BRIEF DESCRIPTION OF THE DRAWING

The invention may be better understood by reference to the accompanying drawing wherein:
The drawing is an isometric view of a diffusion cell for measuring flux of estradiol across mammalian skin.

### DETAILED DESCRIPTION OF THE INVENTION

The present invention relates to pressure-sensitive adhesive sheet materials comprising a backing and a layer of pressure-sensitive adhesive containing estradiol coated thereon. Further, this invention relates to a method of treating conditions associated with estradiol deficiency.

By "treating a condition associated with estradiol deficiency" as used in the instant specification and claims is meant administering a dose of estradiol in an amount and at a rate which eliminates or reduces the occurrence of one or more of the following conditions: osteoporosis, headaches, nausea, depression, hot flashes and any other discomfort that occurs during menopause. By "prolonged period" as used in the instant specification and claims is meant for a period of at least 12 hours.

The adhesives utilized in the practice of the invention should be substantially chemically inert to estradiol. Suitable acrylic adhesive polymers for use in one embodiment of the invention comprise in an amount of 91 to 98 percent by weight, and preferably 94 to 98 percent by weight, respectively, of all monomers in the polymer of a hydrophobic monomeric acrylic or methacrylic acid ester of an alkyl alcohol, the alkyl alcohol containing 4 to 10 carbon atoms. Examples of suitable monomers are those discussed below in connection with the "A Monomer". These adhesive polymers further comprise minor amounts of other monomers such as the "B Monomers" listed below.

Preferred adhesives are acrylic pressure-sensitive adhesive copolymers comprising A and B monomers as follows: Monomer A is a hydrophobic monomeric acrylic or methacrylic acid ester of an alkyl alcohol, the alkyl alcohol containing 4 to 10 carbon atoms, preferably 6 to 10 carbon atoms, more preferably 6 to 8 carbon atoms, and most preferably 8 carbon atoms. Examples of suitable A monomers are n-butyl, n-pentyl, n-hexyl, isoheptyl, n-nonyl, n-decyl, isohexyl, 2-ethyloctyl, isooctyl and 2-ethylhexyl acrylates. The most preferred A monomer is isooctyl acrylate.

Monomer B is a reinforcing monomer selected from the group consisting of acrylic acid; methacrylic acid; alkyl acrylates and methacrylates containing 1 to 3 carbon atoms in the alkyl group; acrylamide; methacrylamide; lower alkyl-substituted acrylamides (i.e. the alkyl group containing 1 to 4 carbon atoms) such as tertiary-butyl acrylamide; diacetone acrylamide; n-vinyl-2-pyrrolidone; vinyl ethers such as vinyl tertiary-butyl ether; substituted ethylenes such as derivatives of maleic anhydride, dimethyl itaconate and monoethyl formate and vinyl perfluoro-n-butyrate. The preferred B monomers are acrylic acid, methacrylic acid, the above-described alkyl acrylates and methacrylates, acrylamide, methacrylamide, and the above-described lower alkyl substituted acrylamides. The most preferred B monomer is acrylamide.

The B monomer in such a copolymer is present in the pressure-sensitive adhesive copolymer in an amount by weight of 2 to 9 percent by weight, and preferably 2 to 6 percent by weight of the weight of all monomers in the copolymer.

In another embodiment of the invention, the acrylic copolymer comprises 60 to 80 percent by weight (and preferably 70 to 80 percent by weight) of the above-mentioned hydrophobic monomeric acrylic or methacrylic acid ester of an alkyl alcohol based on the weight of the monomers in the copolymer; 4 to 9 percent by weight based on the weight of all monomers in the copolymer of a reinforcing monomer selected from the group consisting of acrylic acid, methacrylic acid, an alkyl acrylate or methacrylate containing 1 to 3 carbon atoms in the alkyl group, acrylamide, methacrylamide, a lower alkyl-substituted acrylamide, diacetone acrylamide and N-vinyl-2-pyrrolidone; and 15 to 35 percent by weight (and preferably 15 to 25 percent by weight) of vinyl acetate based on the weight of all monomers in the copolymer. In this embodiment the preferred acrylic or methacrylic acid ester is isooctyl acrylate and the preferred reinforcing monomer is acrylamide. Use of vinyl acetate in preparing the acrylic polymer is a convenient way to reduce the amount of residual monomer in the final preparation as has been described in U.S. Patent No. 4,737,577.

The adhesive copolymers of the above type are known and their method of preparation is well known to those skilled in the art, having been described for example, in U.S. Patent RE 24,906 of Ulricho Since the pressure-sensitive adhesives described above are inherently rubbery and tacky and are suitably heat and light stable, there is no need to add tackifiers or stabilizers. However, such may be added if desired.

The estradiol is present in the adhesive in a pharmaceutically effective amount of from 0.2 to 12 percent by weight of the total weight of the pressure-sensitive adhesive layer of the sheet material, and will preferably be 1 to 5 percent by weight. The most preferred is an amount of 2 to 3.5 percent by weight.

It has been found that the addition of certain skin penetration enhancers significantly enhances the penetration of estradiol in vitro when this phenomena is measured using the hairless mouse skin model as described hereinbelow. Hence, the adhesive sheet material of the invention has an adhesive coating comprising a combination of two or more ingredients in an amount effective to enhance the penetration of estradiol through skin as compared to when said adhesive coating is free of the skin penetration enhancers.

More specifically, the adhesive-coating comprises isopropyl myristate and glyceryl monolaurate as penetration enhancers. In a preferred embodiment, the adhesive-coating additionally comprises ethyl oleate.

The isopropyl myristate will generally be present in an amount of 5 to 20 percent by weight, and preferably 5 to 15 percent by weight, of the total weight of the adhesive coating and the glyceryl monolaurate will generally be present in an amount of to 6 percent, and preferably 2 to 4 percent by weight. When the adhesive-coating additionally contains ethyl oleate, ethyl oleate will generally be present in an amount by weight of 4 to 18 percent, and preferably 5 to 15 percent, based on the weight of the adhesive coating. When ethyl oleate is present the total weight of ethyl oleate and isopropyl myristate will not exceed 25 percent by weight of the adhesive-coating.

A suitable glyceryl monolaurate is that commercially available from Lauricidin Inc. (Monroe, Michigan) under the trade designation Lauricidin (distilled monoglyceride). The backing of the tape may be occlusive, non-occlusive or a breathable film. The backing may be any of the normal materials for pressure-sensitive adhesive tapes such as polyethylene, particularly low-density polyethylene, linear low density polyethylene, high density polyethylene, randomly-oriented nylon fibers, polypropylene, ethylene-vinylacetate copolymer, polyurethane, rayon and the like. The backing should be substantially non-reactive with estradiol.

The presently preferred backing is low density polyethylene. Low density polyethylene backings provide an excellent barrier to loss of estradiol when used with the adhesive formulations of the invention, including those formulations which contain a skin penetration enhancer.

Backings which are layered such as polyethylene-aluminum-polyethylene composites are also suitable.

Although animal skins are known to give significant quantitative differences in drug penetration rates versus human skin, the rank order correlation is generally observed with various drugs (M. J. Bartek and J. A. LaBudde in "Animal Modes in Dermatology", H. Maibach, Ed. Churchill Livingstone, New York, 1975, pp. 103-119). Hairless mouse skin has been recommended as a readily available animal skin for use in diffusion cells with steroids and small molecules (R. B. Stoughton, Arch. Derm., 99, 753 (1969), J. L. Cohen and R. B. Stoughton, J. Invest. Derm., 62, 507 (1974), R. B. Stoughton in "Animal Modes in Dermatology", H. Maibach, Ed., Churchill Livingstone, New York, 1975, pp. 121-131).

In the specific test procedure used herein, skin removed from female hairless mice (available from Jackson Laboratory, Strain HRS/J, age 2-5 months) is used. It is maintained on ice until used. The mouse skin is cut in half and each half is mounted, or the skin is used whole, on a diffusion cell of the type shown in the drawing. The cell is modeled after those described in the literature (e.g. J. L. Cohen, R. B. Stoughton, J. Invest. Derm. 62 507 (1974) and R. B. Stoughton, Arch. Derm. 99, 753 (1964). As shown in the figure, the mouse or human skin (20) is mounted epidermal side up between the upper and lower portions of the cell (21) and (22), which are held together by means of a ball joint clamp (23). The cell below the skin is filled with 30% N-methyl-2-pyrrolidone in water to act as the "acceptor" fluid. The acceptor fluid is stirred using a magnetic stirring bar (24) and a magnetic stirrer (not illustrated). The sampling port (25) is stoppered except when in use.

A known amount of a formulation to be evaluated is applied to the epidermal (upper) side of the skin in a uniform layer as follows: The desired area and weight of a sheet material formulation is accurately determined so that the amount of adhesive applied to the cell can be accurately determined. The sheet material is applied to the skin already mounted on the diffusion cell and pressed to cause uniform contact to the skin.

The cell is then placed in a constant temperature (31 to 33 C) constant humidity chamber (generally maintained at a humidity between 40 and 50%, preferably about 50%) and kept there throughout the experiment. The chamber utilizes a heat exchanger coupled to a constant temperature bath, with a fan to circulate air. A saturated calcium nitrate solution is used to maintain the humidity. The acceptor fluid is stirred by means of a magnetic stirring bar throughout the experiment to assure a uniform sample and a reduced diffusion layer on the dermal side of the skin. The acceptor fluid is removed at specified time intervals and fresh fluid is immediately added to replace the withdrawn fluid. The withdrawn aliquots are analyzed for drug content by conventional high pressure liquid chromatograpy and the cumulative amount of the drug penetrating the skin is calculated. Plots of the cumulative drug penetration as a function of time give a profile of the drug flux measured in microg/cm²/hour.

The use of other skin such as human skin in the above apparatus has confirmed the utility of the formulations of the invention.

The sheet materials of the present invention are preferably prepared by combining dry adhesive, estradiol and the skin penetration enhancers with an organic solvent. Preferred organic solvents are methanol and ethyl acetate. The total solids content will be in the range of 15 to 40% and preferably 20 to 35%. The resulting mixture is shaken at a high speed until a homogenous solution is obtained and then allowed to stand to dissipate air bubbles. The resulting formulation may be wet cast or coated by wet-cast or knife coating techniques to provide a predetermined uniform thickness of the wet adhesive formulation onto a suitable release liner. This sheet is then dried and laminated onto a backing material using conventional methods. Suitable release liners are known silicone-type release liners such as that available under the trade designation Daubert 164, from Daubert Co. which are coated onto polyester film. The adhesive coated sheet material of the invention may be in the form of a tape, a patch, a sheet, a dressing or other forms known to the art as will be apparent to one skilled in the art. Preferably, the adhesive coated sheet material will contain 0.2 to 7.0 mg, and preferably 1.0 to 2.0 mg, of estradiol per 5 cm² of the sheet material. Further, the sheet material will generally be 1 to 40 cm² , and preferably 10 to 30 cm², in dimension.

Generally, a transdermal patch of the invention will be applied to the skin of a mammal (preferably a human) and will be replaced with a fresh patch as required to maintain the therapeutic effect. Those skilled in the art may easily determine the frequency at which the patches of the invention should be replaced to achieve the desired therapeutic effect.

The following examples are provided to illustrate the invention, but are not intended to be limiting thereof. Parts and percentages are by weight unless otherwise specified. Flux rates are expressed in units of micrograms of estradiol per cm² for the time period specified in the example. Each result represents the average value of several (e.g., 3 to 5) independent determinations.

### Inherent Viscosity Measurement

In the examples which follow, it is useful to refer to the molecular weight of the adhesive polymer used in the adhesive formulations. The comparative molecular weights are determined by measuring the viscosity of dilute solutions of the adhesives prepared according to these teachings.

The inherent viscosity values which are reported in the examples which follow were obtained by the conventional method used by those skilled in the art. The measurement of the viscosity of dilute solutions of the adhesive, when compared to controls run under the same conditions, clearly demonstrates the relative molecular weights. It is the comparative values which are significant and absolute figures are not required. In the examples, the inherent viscosity values were obtained using a Cannon-Fenske #50 viscometer in a water bath controlled at 25°C to measure the flow time of 10 ml of a polymer solution. The examples and controls being run for comparison were run under identical conditions. The test procedure followed and the apparatus used are explained in detail in the Textbook of Polymer Science, F. W. Billmeyer, Wiley-Interscience, 2nd Edition, 1971 under: Polymer chains and their characterization, D. Solution Viscosity and Molecular Size, pages 84 and 85.

### Preparation of Isooctyl Acrylate: Acrylamide (94:6) Copolymer

To a 114 gram narrow-mouth glass bottle were added: 127.84 g. isooctyl acrylate, 8.6 g. acrylamide, 0.41 g. benzoyl peroxide, 237.6 g. ethyl acetate and 26.4 g. methyl alcohol. The solution was purged for two minutes with nitrogen at a flow rate of one liter per minute. The bottle was sealed and placed in a rotating water bath at 55°C for twenty four hours to effect essentially complete polymerization. The polymer was diluted with ethyl acetate/methyl alcohol (90/10) to 28.4% solids and had a measured inherent viscosity of 1.02 dl/g. in ethyl acetate at a concentration of 0.15 g/dl. Its Brookfield viscosity was 9,120 centipoise.

### Preparation of Isooctyl Acrylate: Acrylamide (95:5) Copolymer

The procedures above were repeated, this time employing 152.00 g. isooctyl acrylate, 8.0 g. acrylamide, 0.48 g. benzoyl peroxide, 216.0 g. ethyl acetate and 24.0 g. methyl alcohol. The resulting polymer was diluted with the ethyl acetate/methyl alcohol mixtures to 29.38% solids. The polymer had a measured inherent viscosity of 1.32 dl/g in ethyl acetate at a concentration of 0.15 g/dl. Its Brookfield viscosity was 9,900 centipoise.

A 25-30 percent solids solution of the above isooctyl acrylate:acrylamide (94:6) adhesive copolymer or the above isooctyl acrylate: acrylamide (95:5) adhesive copolymer in ethyl acetate/methanol (90:10) was coated onto a 2 sided release liner using a knife-coater and coating at 508µm (20 mils) in thickness. The adhesive-coated laminate was dried first at 82.2°C (180°F) for 3 minutes and then at 115.5°C (240°F) for 3 minutes. The dried adhesive coating was then stripped off the release liner and placed into a salt glass bottle. The foregoing procedure results in a reduction of the amount of residual monomer which may be contained in the adhesive copolymer.

### Preparation of Isooctyl Acrylate: Acrylamide: Vinyl Acetate (75:5:20) Copolymer

The procedures above were repreated this time employing 120.0 g. isooctyl acrylate, 8.0 g. acrylamide, 32.0 g. vinyl acetate, 0.32 g. benzoyl peroxide, 216.0 g. ethyl acetate and 24.0 g. methyl alcohol. The resulting polymer was diluted with the ethyl acetate/methyl alcohol mixture to 21.52% solids. The adhesive polymer had a measured inherent viscosity of 1.40 dl/g in ethyl acetate at a concentration of 0.15 g/dl. Its Brookfield viscosity was 2,300 centipoise.

### Example 1

A mixture of 200.62 g of 95:5 isoctyl acrylate:acrylamide adhesive copolymer, 33.75 g of isopropyl myristate, 8.75 g of glyceryl monolaurate, 6.88 g of estradiol USP, 525.00 g of ethyl acetate and 58.33 g of methanol was placed in a jar. The jar was placed on a platform shaker and shaken for about 18 hours. The formulation was allowed to stand until all the air bubbles had dissipated. The formulation was coated at a thickness of 0.56 mm (0.022 inches) onto a silicone coated 127µm(5 mil) liner. The laminate was oven dried for 2 minutes at 51.6°C (125°F) for 2 minutes at 85°C (185°F) and for 2 minutes at 113°C (235°F) (too vigorous conditions for drying may result in loss of a major amount of isopropyl myristate). The resulting adhesive coating contained 80.25 percent 95:5 isooctyl acrylate:acrylamide adhesive copolymer, 13.50 percent isopropyl myristrate, 3.50 percent glyceryl monolaurate and 2.75 percent estradiol. The material was allowed to cool and was then laminated onto the corona treated surface of 76.2µm (3 mil) low density polyethylene backing. The laminate was die cut into 2 cm² patches. Penetration through hairless mouse skin was measured using the diffusion apparatus and method described above. The acceptor fluid was 30% N-methyl-2-pyrrolidone in water. Three independent determinations were carried out. The average penetration in 24 hours was 74 micrograms/cm².

### Examples 2-4

Using the general method of Example 1 the formulations shown in Table 1 were prepared and the penetration through hairless mouse skin measured. The acceptor fluid was 30% N-methyl-2-pyrrolidone in water. Patches which measured 2 cm² were employed.

**Table 1**

| Formulation | | Penetration Micrograms/cm² in 24 hrs |
|---|---|---|
| 2.75% | estradiol | |
| 3.50% | glyceryl monolaurate | |
| 13.50% | isopropyl myristate | |
| 80.25% | isooctyl acrylate: acrylamide copolymer (94:6) | 75 |
| 2.75% | estradiol | |
| 3.50% | glyceryl monolaurate | |
| 10.60% | isopropyl myristate | |
| 5.30% | ethyl oleate | |
| 77.85% | isooctyl acrylate: acrylamide copolymer (95:5) | 87 |
| 2.75% | estradiol | |
| 3.50% | glyceryl monolaurate | |
| 10.60% | isopropyl myristate | |
| 5.30% | ethyl oleate | |
| 77.85% | isooctyl acrylate: acrylamide copolymer (94:6) | 87 |

### Example 5

A mixture of 23.31 g of isooctyl acrylate:acrylamide:vinyl acetate adhesive copolymer, 21.66% solids in 90:10 ethyl acetate:methanol, 0.184 g of estradiol USP, 0.8035 g of isopropyl myristate, 0.2402 g of glyceryl monolaurate and 0.4130 g of ethyl oleate was placed in a jar. The jar was placed on a platform shaker and shaken for about 20 hours. The formulation was allowed to stand until air bubbles had dissipated. The formulation was coated at a thickness of 0.56mm(0.022 inches) onto a 127µm (5 mil) Daubert 164Z release liner. The laminate was oven dried for 4 min. at 51.6°C (125°F,) for 2 minutes at 85°C (185°F) and for 1 minute at 107°C (225°F). The resulting adhesive coating contained 75.5 percent 75:5:20 isooctyl acrylate:acrylamide:vinyl acetate adhesive copolymer, 2.75 percent estradiol, 12,0 percent isopropyl myristate, 6.2 percent ethyl oleate and 3.6 percent glyceryl monolaurate. The material was then laminated onto the corona treated surface of a 76.2 m (3 mil) low density polyethylene film and die cut into 5.07 cm² patches. Penetration through hairless mouse skin was measured. The acceptor fluid was 30% N-methyl-2-pyrrolidone in water. Three independent determinations were made. The average amount penetrating in 24 hours was 84 micrograms/cm².

### Examples 6-8

Using the general method of Example 5 the formulations shown in Table 2 were prepared and the penetration through hairless mouse skin measured. The adhesive used was an isooctyl acrylate:acrylamide:vinyl acetate 75:5:20 copolymer. The acceptor fluid was 30% N-methyl-2-pyrrolidone in water. Patches measuring 5.07 cm² were employed.

**Table 2**

| Formulation | Penetration Micrograms/cm² in 24 hrs |
|---|---|
| 2.75% estradiol | |
| 14.0% isopropyl myristate | |
| 7.4% ethyl oleate | |
| 3.5% glyceryl monolaurate | |
| 72.7% adhesive | 112 |
| 2.74% estradiol | |
| 6.1% isopropyl myristate | |
| 12.4% ethyl oleate | |
| 3.5% glyceryl monolaurate | |
| 75.2% adhesive | 98 |
| 2.76% estradiol | |
| 7.0% isopropyl myristate | |
| 14.1% ethyl oleate | |
| 3.5% glyceryl monolaurate | |
| 72.6% adhesive | 130 |

## Claims

1. An adhesive-coated sheet material comprising:
a) a flexible backing; and
b) a pressure-sensitive adhesive coating contiguously adhered to one surface of said backing and comprising a homogeneous mixture of:
i) an acrylic copolymer comprising 91 to 98 percent by weight of a hydrophobic monomeric acrylic or methacrylic acid ester of an alkyl alcohol based on the weight of all monomers in said copolymer, the alkyl alcohol containing 4 to 10 carbon atoms;
ii) estradiol in an amount by weight of 0.2 to 12 percent of the total weight of said adhesive coating; and
iii) a skin penetration enhancer combination comprising isopropyl myristate and glyceryl monolaurate in amounts of 5 to 20 percent and 1 to 6 percent by weight, respectively, based on the weight of said adhesive-coating, with the relative amounts being selected so as to enhance the penetration of said estradiol through skin as compared to when said adhesive coating is free of said skin penetration enhancers;
said sheet material being further characterized in that over a prolonged period it adheres suitably to skin and provides for substantially continuous transdermal delivery of estradiol to a subject in an amount which is therapeutically effective for treating a condition associated with estradiol deficiency.

2. An adhesive-coated sheet material according to Claim 1, wherein said pressure-sensitive acrylic adhesive copolymer comprises A and B monomers as follows:
A is a hydrophobic monomeric acrylic or methacrylic acid ester of an alkyl alcohol, the alkyl alcohol containing 4 to 10 carbon atoms, said A monomer being present in an amount by weight of 91 to 98 percent of the total weight of all monomers in said copolymer; and
B is a reinforcing monomer selected from the group consisting of acrylic acid, methacrylic acid, an alkyl acrylate or methacrylate containing 1 to 3 carbon atoms in the alkyl group, acrylamide, methacrylamide, a lower alkyl-substituted acrylamide, diacetone acrylamide, N-vinyl-2-pyrrolidone, a vinyl ether, a substituted ethylene and a vinyl ester, the B monomer being present in an amount by weight of 2 to 9 percent of the total weight of all monomers in said copolymer.

3. An adhesive-coated sheet material according to Claim 1, wherein said adhesive copolymer comprises said acrylic or methacrylic acid ester in an amount of 94 to 98 percent by weight.

4. An adhesive-coated sheet material according to Claim 2, wherein said adhesive copolymer comprises isooctyl actylate as the A monomer and acrylamide as the B monomer.

5. An adhesive-coated sheet material according to any preceding claim wherein the amount of estradiol in said adhesive coating is 1 to 5 percent by weight of said adhesive coating.

6. An adhesive-coated sheet material according to any of claims 1-4 wherein the amount of estradiol in said adhesive coating is 2 to 3.5 percent by weight of said adhesive coating.

7. An adhesive-coated sheet material according to any preceding claim wherein said isopropyl myristate and glyceryl monolaurate are present in amounts by weight of 5 to 15 percent and 2 to 4 percent, respectively, based on the weight said adhesive coating.

8. An adhesive-coated sheet material according to any preceding claim wherein said skin penetration enhancer combination further comprises 4 to 18 percent by weight of ethyl oleate based on the weight of said adhesive coating, and wherein the total amount by weight of isopropyl myristate and ethyl oleate is less than 25 percent based on the weight of said adhesive coating.

9. An adhesive-coated sheet material comprising:
a) a flexible backing; and
b) a pressure-sensitive adhesive coating contiguously adhered to one surface of said backing and comprising a homogeneous mixture of:
i) an acrylic copolymer comprising (1) 60 to 80 percent by weight of a hydrophobic monomeric acrylic or methacrylic acid ester of an alkyl alcohol based on the weight of all monomers in said copolymer, the alkyl alcohol containing 4 to 10 carbon atoms; (2) 4 to 9 percent by weight based on the weight of all monomers in said copolymer of a reinforcing monomer selected from the group consisting of acrylic acid, methacrylic acid, an alkyl acrylate or methacrylate containing 1 to 3 carbon atoms in the alkyl group, acrylamide, methacrylamide, a lower alkyl-substituted acrylamide, diacetone acrylamide, and N-vinyl-2-pyrrolidone; and (3) 15 to 35 percent by weight of vinyl acetate based on the weight of all monomers in said copolymer;
ii) estradiol in an amount by weight of 0.2 to 12 percent of the total weight of said adhesive coating; and
iii) a skin penetration enhancer combination comprising isopropyl myristate and glyceryl monolaurate in amounts of 5 to 20 percent and 1 to 6 percent by weight, respectively, based on the weight of said adhesive coating with the relative amounts being selected so as to enhance the penetration of said estradiol through skin as compared to when said adhesive coating is free of said skin penetration enhancers;
said sheet material being further characterized in that over a prolonged period it adheres suitably to skin and provides substantially continuous transdermal delivery of estradiol to a subject in an amount which is therapeutically effective for treating a condition associated with estradiol deficiency.

10. An adhesive-coated sheet material according to Claim 9 wherein said ester is isooctyl acrylate and said reinforcing monomer is acrylamide.

11. An adhesive-coated sheet material according to any one of claims 9-10 wherein the amount of estradiol in said adhesive coating is 1 to 5 percent by weight of said adhesive coating.

12. An adhesive-coated sheet material according to any one of claims 9-10 wherein the amount of estradiol in said adhesive coating is 2 to 3.5 percent by weight of said coating.

13. An adhesive-coated sheet material according to any one of claims 9-12 wherein said isopropyl myristate and glyceryl monolaurate are present in amounts by weight of 5 to 15 percent and 2 to 4 percent, respectively, based on the weight of said adhesive coating.

14. An adhesive-coated sheet material according to any one of claims 9-13 wherein said skin penetration enhancer combination further comprises 4 to 18% by weight of ethyl oleate based on the weight of said adhesive coating, and wherein the total amount by weight of isopropyl myristate and ethyl oleate is less than 25% by weight based on the weight of said adhesive coating.

## Patentansprüche

1. Klebstoffüberzogenes Blattmaterial mit
a) einem flexiblen Rücken und
b) einem eine Oberfläche des Rückens haftend berührenden Haftkleberüberzug, der wenigstens teilweise aus einem homogenen Gemisch aus
i) einem Acrylcopolymer mit auf das Gewicht aller Monomere des Copolymers bezogen 91 bis 98 Gew.-% eines hydrophoben monomeren Acryl- oder Methacrylsäureesters eines 4 bis 10 Kohlenstoffatome besitzenden Alkylalkohols,
ii) Östradiol in einer Menge von 0,2 bis 12% des Gesamtgewichts des Haftkleberüberzuges und
iii) einer Hautpenetrationsverstärkerkombination mit Isopropylmyristat und Glycerylmonolaurat in auf das Gewicht des Haftkleberüberzuges bezogenen Menge von 5 bis 20 Gew.-% bzw. 1 bis 6 Gew.-% besteht, wobei das Mengenverhältnis so gewählt ist, daß das Östradiol die Haut leichter durchdringt als aus dem von den Hautpenetrationsverstärkern freien Überzug,
besteht,
wobei das Blattmaterial ferner dadurch gekennzeichnet ist, daß es während eines längeren Zeitraums an der Haut in geeigneter Weise festklebt und eine im wesentlichen kontinuierliche transdermale Abgabe von Östradiol an eine Trägerin in einer Menge ermöglich, die zur Behandlung eines einem Östradiolmangel zugeordneten Zustandes therapeutisch wirksam ist.

2. Klebstoffüberzogenes Blattmaterial nach Anspruch 1, in dem das Haftkleber-Acrylcopolymer die nachstehend angegebenen Monomere A und B enthält:
A ist ein hydrophober monomerer Acryl- oder Methacrylsäureester eines 4 bis 10 Kohlenstoffatome besitzenden Alkylalkohols und ist in einer Menge von 91 bis 98 Gew.-% des Gesamtgewichts aller Monomere des Copolymers vorhanden, und
B ist ein Verstärkungsmonomer, das aus der Gruppe ausgewählt ist, die aus Acrylsäure, Methacrylsäure, einem Alkylacrylat oder -methacrylat mit 1 bis 3 Kohlenstoffatomen in der Alkylgruppe, Acrylamid, Methacrylamid, einem mit einem niederen Alkyl substituierten Acrylamid, Diacetonacrylamid, N-Vinyl-2-pyrrolidon, einem Vinylether, einem substituierten Ethylen und einem Vinylester besteht, wobei das Monomer in einer Menge von 2 bis 9 Gew.-% des Gesamtgewichts aller Monomere des Copolymers vorhanden ist.

3. Klebstoffüberzogenes Blattmaterial nach Anspruch 1, in dem das Haftkleber-Copolymer den Acryl- oder Methacrylsäureester in einer Menge von 94 bis 98 Gew.-% enthält.

4. Klebstoffüberzogenes ßlattmaterial nach Anspruch 2, in dem das Haftkleber-Copolymer Isooctylacrylat als Monomer A und Acrylamid als Monomer B enthält.

5. Klebstoffüberzogenes Blattmaterial nach einem der vorhergehenden Ansprüche, in dem der Haftkleberüberzug das Östradiol in einer Menge von 1 bis 5 Gew.-% des Haftkleberüberzuges enthält.

6. Klebstoffüberzogenes Blattmaterial nach einem der Ansprüche 1 bis 4, in dem der Haftkleberüberzug das Östradiol in einer Menge von 2 bis 3,5 Gew.-% des Haftkleberüberzuges enthält.

7. Haftklberüberzogenes Blattmaterial nach einem der vorhergehenden Ansprüche, in dem das Isopropylmyristat und das Glycerylmonolaurat in auf das Gewicht der haftkleberüberzogenen Mengen von 5 bis 15 Gew.-% bzw. von 2 bis 4 Gew.-% vorhanden sind.

8. Klebstoffüberzogenes Blattmaterial nach einem der vorhergehenden Ansprüche, in dem die Hautpenetrationsverstärkekombination ferner auf das Gewicht des Haftkleberüberzuges bezogen 4 bis 18 Gew.-% Ethyloleat enthält und die Gesamtmenge an Isopropylmyristat und Ethyloleat weniger auf das Gewicht des Haftkleberüberzuges bezogen weniger als 25 Gew.-% beträgt.

9. Klebstoffüberzogenes Blattmaterial mit
a) einem flexiblen Rücken und
b) einem eine Oberfläche des Rückens haftend berührenden Haftkleberüberzug, der wenigstens teilweise aus einem homogenen Gemisch aus
i) einem Acrylcopolymer mit auf das Gewicht aller Monomere des Copolymers bezogen (1) 60 bis 80 Gew.-% eines hydrophoben monomeren Acryl- oder Methacrylsäureesters eines 4 bis 10 Kohlenstoffatome besitzenden Alkylalkohols, (2) auf das Gewicht aller Monomere des Copolymers bezogen 4 bis 9 Gew.-% eines Verstärkungsmonomers, das aus der Gruppe ausgewählt ist, die aus Acrylsäure, Methacrylsäure, einem Alkylacrylat oder -methacrylat mit 1 bis 3 Kohlenstoffatomen in der Alkylgruppe, Acrylamid, Methacrylamid, einem mit einem niederen Alkyl substituierten Acrylamid und Diacetonacrylamid besteht, und (3) auf das Gewicht aller Monomere des Copolymers bezogen 15 bis 35 Gew.-% Vinylacetat,
ii) Östradiol in einer Menge von 0,2 bis 12% des Gesamtgewichts des Haftkleberüberzuges und
iii) einer Hautpenetrationsverstärkerkombination mit Isopropylmyristat und Glycerylmonolaurat in auf das Gewicht des Haftkleberüberzuges bezogenen Mengen von 5 bis 20 Gew.-% bzw. 1 bis 6 Gew.-% besteht, wobei das Mengenverhältnis so gewählt ist, daß das Östradiol die Haut leichter durchdringt als aus dem von den Hautpenetrationsverstärkern freien Überzug,
besteht,
wobei das Blattmaterial ferner dadurch gekennzeichnet ist, daß es während eines längeren Zeitraums an der Haut in geeigneter Weise festklebt und eine im wesentlichen kontinuierliche transdermale Abgabe von Östradiol an eine Trägerin in einer Menge ermöglicht, die zur Behandlung eines einem Östradiolmangel zugeordneten Zustandes therapeutisch wirksam ist.

10. Klebstoffüberzogenes Blattmaterial nach Anspruch 9, in dem der Ester Isooctylacrylat und das Verstärkungsmonomer Acrylamid ist.

11. Klebstoffüberzogenes Blattmaterial nach Anspruch 9 oder 10, in dem der Haftkleberüberzug das Östradiol in einer Menge von 1 bis 5 Gew.-% des Haftkleberüberzuges enthält.

12. Klebstoffüberzogenes Blattmaterial nach Anspruch 9 oder 10, in dem der Haftkleberüberzug das Östradiol in einer Menge von 2 bis 3,5 Gew.-% des Haftkleberüberzuges enthält.

13. Klebstoffüberzogenes Blattmaterial nach einem der Ansprüche 9 bis 12, in dem das Isopropylmyristat und das Glycerylmonolaurat in auf das Gewicht der haftkleberüberzogenen Mengen von 5 bis 15 Gew.-% bzw. von 2 bis 4 Gew.-% vorhanden sind.

14. Klebstoffüberzogenes Blattmaterial nach einem der Ansprüche 9 bis 13, in dem die Hautpenetrationsverstärkerkombination ferner auf das Gewicht des Haftkleberüberzuges bezogen 4 bis 18 Gew.-% Ethyloleat enthält und die Gesamtmenge an Isopropylmyristat und Ethyloleat weniger auf das Gewicht des Haftkleberüberzuges bezogen weniger als 25 Gew.-% beträgt.

## Revendications

1. Matière en feuille revêtue d'adhésif comprenant :
a) un support flexible ; et
b) un revêtement adhésif sensible à la pression ou autocollant adhérant de façon contiguë à une surface du support et comprenant un mélange homogène :
i) d'un polymère acrylique comprenant 91 à 98 % en poids d'un ester d'acide acrylique ou méthacrylique monomère hydrophobe d'un alcool alkylique par rapport au poids de tous les monomères dans le polymère, l'alcool alkylique contenant 4 à 10 atomes de carbone ;
ii) d'oestradiol en une quantité pondérale de 0,2 à 12 % du poids total du revêtement adhésif ; et
iii)d'une combinaison d'activateurs de pénétration cutanée comprenant du myristate d'isopropyle et du monolaurate de glycéryle en des quantités respectivement de 5 à 20 % et de 1 à 6 % en poids par rapport au poids du revêtement adhésif, les quantités relatives étant choisies de manière à activer la pénétration de l'oestradiol à travers la peau en comparaison du cas où le revêtement adhésif est exempt de ces activateurs de pénétration cutanée ;
cette matière en feuille étant de plus caractérisée en ce que sur une période prolongée elle adhère de façon appropriée à la peau et permet une distribution transdermique sensiblement continue d'oestradiol à un sujet en une quantité qui est thérapeutiquement efficace pour le traitement d'un état associé à une carence en oestradiol.

2. Matière en feuille revêtue d'adhésif suivant la revendication 1, dans laquelle le copolymère adhésif acrylique sensible à la pression comprend des monomères A et B de la façon suivante :
A représente un ester d'acide acrylique ou méthacrylique monomère hydrophobe d'un alcool alkylique, l'alcool alkylique contenant 4 à 10 atomes de carbone, le monomère A susdit étant présent en une quantité en poids de 91 à 98% du poids total de tous les monomères dans le copolymère ; et
B représente un monomère de renforcement choisi dans le groupe comprenant l'acide acrylique, l'acide méthacrylique, un acrylate ou méthacrylate d'alkyle contenant de 1 à 3 atomes de carbone dans le groupe alkyle, l'acrylamide, le méthacrylamide, un acrylamide à substitution d'alkyle inférieur, le diacétone acrylamide, la N-vinyl-2-pyrrolidone, un éther vinylique, un éthylène substitué et un ester vinylique, le monomère B étant présent en une quantité en poids de 2 à 9 % du poids total de tous les monomères dans le copolymère.

3. Matière en feuille revêtue d'adhésif suivant la revendication 1, dans laquelle le copolymère adhésif comprend l'ester d'acide acrylique ou méthacrylique en une quantité de 94 à 98 % en poids.

4. Matière en feuille revêtue d'adhésif suivant la revendication 2, dans laquelle le copolymère adhésif comprend de l'acrylate d'isooctyle comme monomère A et de l'acrylamide comme monomère B.

5. Matière en feuille revêtue d'adhésif suivant l'une quelconque des revendications précédentes, dans laquelle la quantité d'oestradiol dans le revêtement adhésif est de 1 à 5 % en poids du revêtement adhésif.

6. Matière en feuille revêtue d'adhésif suivant l'une quelconque des revendications 1 à 4, dans laquelle la quantité d'oestradiol dans le revêtement adhésif est de 2 à 3,5 % en poids du revêtement adhésif.

7. Matière en feuille revêtue d'adhésif suivant l'une quelconque des revendications précédentes, dans laquelle le myristate d'isopropyle et le monolaurate de glycéryle sont présents en des quantités en poids respectivement de 5 à 15 % et 2 à 4 % par rapport au poids du revêtement adhésif.

8. Matière en feuille revêtue d'adhésif suivant l'une quelconque des revendications précédentes, dans laquelle la combinaison d'activateurs de pénétration cutanée comprend de plus 4 à 18 % en poids d'oléate d'éthyle par rapport au poids du revêtement adhésif et dans laquelle la quantité totale en poids de myristate d'isopropyle et d'oléate d'éthyle est inférieure à 25 % en poids par rapport au poids du revêtement adhésif.

9. Matière en feuille revêtue d'adhésif comprenant :
a) une support flexible ; et
b) un revêtement adhésif sensible à la pression ou autocollant adhérant de façon contiguë à une surface du support et comprenant un mélange homogène :
i) d'un copolymère acrylique comprenant (1) 60 à 80 % en poids d'un ester d'acide acrylique ou méthacrylique monomère hydrophobe d'un alcool alkylique par rapport au poids de tous les monomères dans le copolymère, l'alcool alkylique contenant 4 à 10 atomes de carbone ; (2) 4 à 9 % en poids par rapport au poids de tous les monomères dans le copolymère d'un monomère de renforcement choisi dans le groupe comprenant l'acide acrylique, l'acide méthacrylique, un acrylate ou méthacrylate d'alkyle contenant 1 à 3 atomes de carbone dans le groupe alkyle, l'acrylamide, le méthacrylamide, un acrylamide à substitution d'alkyle inférieur, le diacétone acrylamide et la N-vinyl-2-pyrrolidone ; et (3) 15 à 35 % en poids d'acétate de vinyle par rapport au poids de tous les monomères dans le copolymère ;
ii) d'oestradiol en une quantité pondérale de 0,2 à 12 % en poids du poids total du revêtement adhésif ; et
iii) d'une combinaison d'activateurs de pénétration cutanée comprenant du myristate d'isopropyle et du monolaurate de glycéryle en des quantités respectives de 5 à 20 % et de 1 à 6 % en poids par rapport au poids du revêtement adhésif, les quantités relatives étant choisies de manière à activer la pénétration de l'oestradiol à travers la peau en comparaison du cas où le revêtement adhésif est exempt des activateurs de pénétration cutanée ;
ladite matière en feuille étant de plus caractérisée en ce que sur une période prolongée elle adhère de façon appropriée à la peau et produit une distribution transdermique sensiblement continue d'oestradiol à un sujet en une quantité qui est thérapeutiquement efficace pour le traitement d'un état associé à une carence en oestradiol.

10. Matière en feuille revêtue d'adhésif suivant la revendication 9, dans laquelle l'ester est de l'acrylate d'isooctyle et le monomère de renforcement est de l'acrylamide.

11. Matière en feuille revêtue d'adhésif suivant l'une ou l'autre des revendications 9 et 10, dans laquelle la quantité d'oestradiol dans le revêtement adhésif est de 1 à 5 % en poids du revêtement adhésif.

12. Matière en feuille revêtue d'adhésif suivant l'une ou l'autre des revendications 9 et 10, dans laquelle la quantité d'oestradiol dans le revêtement adhésif est de 2 à 3,5 % en poids du revêtement.

13. Matière en feuille revêtue d'adhésif suivant l'une quelconque des revendications 9 à 12, dans laquelle le myristate d'isopropyle et le monolaurate de glycéryle sont présents en des quantités en poids respectivement de 5 à 15 % et de 2 à 4 % par rapport au poids du revêtement adhésif.

14. Matière en feuille revêtue d'adhésif suivant l'une quelconque des revendications 9 à 13, dans laquelle le combinaison d'activateurs de pénétration cutanée comprend de plus 4 à 18 % en poids d'oléate d'éthyle par rapport au poids du revêtement adhésif et dans laquelle la quantité totale en poids de myristate d'isopropyle et d'oléate d'éthyle est inférieure à 25 % en poids par rapport au poids du revêtement adhésif.
